# EUROPEAN PATENT APPLICATION

(11) **EP 0 643 305 A2**
(43) Date of publication of application: **15.03.1995**
(21) Application number: 94306655.5
(22) Date of filing: 12.09.1994
(51) Int. Cl.: G01N 33/543

(54) **Undercoating of solid phase surfaces and direct coating method**

(30) Priority: 13.09.1993 US 120996
(71) Applicant: Ciba Corning Diagnostics Corp., Medfield Massachusetts 02052 (US)
(72) Inventor: Chen, Shin-Yih, Wellesley, Massachussetts 02181 (US)
(74) Representative: Froud, Clive

(57) **Abstract**

An intermediate for use in forming a reagent for analysis of biological solutions is provided comprising a solid phase and an undercoating of inert material covalently or absorptively coated onto the solid phase. Additionally, a reagent is provided in which a binding partner of a reactant to be captured in a test assay is coupled to the undercoating. The solid phase may comprise superparamagnetic particles typically having highly irregular surface structures including small pores which consume binding partner in traditional loading procedures. In the inventive reagent, undercoating fills such small pores and covers the solid phase surface, reducing non-specific binding and allowing tailored loading of binding partner onto the solid phase.

## Description

The present invention relates generally to reagent materials for use in test assays, and more specifically to an inert undercoating provided on insoluble support surfaces used in analytical assays, onto which at least one binding partner of at least one analyte may be coupled to form a reagent.

In the field of biology, qualitative and/or quantitative analyses often exploit the principle that a particular species may adhere specifically to another, such adherence taking the form of enzyme/substrate interaction, antibody/antigen or hapten specific binding, binding or annealing of complimentary base sequences, or the like. In the field of immunology such analyses commonly take the form of immunoassays in which a binding partner of a reactant (analyte) in a fluid sample is attached to a solid phase insoluble in the fluid sample, the analyte being allowed to form a complex with the binding partner to determine its presence and/or concentration.

Solid phase materials and/or surfaces to which binding partners are typically attached in such assays include glass or polymer beads, slides, titre plates, test tube walls, optical waveguides, metal particles, and the like. A variety of methods for attaching the binding partner to the surface are known. According to one of the most common methods, binding partners are covalently attached to silane-coated surfaces via glutaraldehyde linkage.

Presently-available techniques for attachment, or loading, of binding partners onto solid phase surfaces include certain drawbacks. Protein binding partners directly linked to surfaces, for example via glutaraldehyde, are often linked through excessive numbers of covalent bonds involving amino acids, the result of which may be partial or total denaturing of the protein, and resultant decrease in its effectiveness as a binding partner in a test assay. Additionally, it is often advantageous to only partially coat a surface with a binding partner in an attempt to tailor the reactivity of the surface, leaving a significant amount of uncovered surface area. The uncovered surface area is often of a chemical nature that facilitates non-specific binding of the analyte to the surface, necessarily imparting inaccuracy to an assay. Inexpensive, immunologically inert "blocking agents" may be added to such surfaces so as to coat uncovered surface area, and thus to reduce or eliminate non-specific binding. However, such blocking agents may become detached from the surface over time, adding not only inaccuracy but inconsistency to an assay. Blocking agents may also actually cover the binding partner, reducing the binding capacity per weight of binding partner, a serious consideration when the binding partner is expensive or tedious to prepare.

Such complications must be considered in assays in which magnetic particles are employed as the solid phase. Magnetic particles serve as a high-surface-area solid phase material which may be conveniently separated from solution using a magnetic field. Such magnetic particles typically have a highly irregular surface area, including many pores. Larger ones of such pores and irregularities advantageously increase the surface area of magnetic particles. However, the smaller of such pores offer the following complication. When a quantity of binding partner is loaded onto the surface of magnetic particles, much binding partner material is typically consumed in the filling of small pores. To reduce non-specific binding, a large excess of binding partner may have to be loaded onto the solid phase such that all small pores are filled and a consistent layer of binding partner exists on the surface and essentially no uncovered surface exists, but this procedure is disadvantageous for- several reasons. First, as noted above, binding partner is typically precious material and the procedure is wasteful. Second, it is often desirable to load the solid phase to a particular degree less than the maximum degree of loading, that is, to tailor the degree of loading for a particular analysis, and this is not feasible according to this procedure. Third, the procedure often results in inconsistent loading between batches of solid phase materials, dependent somewhat on the particular surface characteristics of the particular batches.

When an analysis involves the loading of a ligand such as a hapten onto a surface, a highly irregular surface area is especially disadvantageous as the ligand is typically rendered invisible to a reactant (an analyte) to be captured by the binding partner to a large extent. Therefore, it is common to attach a ligand to a carrier molecule or conjugate prior to surface loading, but this additional step adds variability, hence inconsistency, to the procedure. Additionally, ligand is wasted in this procedure as not all ligand/carrier conjugates adhere to the surface oriented such that the binding sites of the ligand faces in a direction so as to be available for complexation, or specific reaction with a reactant to be captured.

U.S. Patent No. 4,478,946 describes an immuno-reactant bonded to a cross-linked film enveloping a carrier body. The described cross-linked film comprises portions of the immuno-reactant itself, for example antigen and antibody. A preferred carrier body comprises a solid, un-silanized, non-porus glass bead. The film is not covalently attached to the surface, but is cross-linked so as to envelop the non-porus surface of the body.

Thus, a need exists in the art for binding partner surface loading techniques in which non-specific binding is minimized, binding partner material is conserved, the binding activity of a particular surface may be controlled, the surfaces loaded reproducibly from batch to batch, and in which ligand may be loaded onto highly irregular surfaces economically, conveniently and reproducibly.

Accordingly, it is a general purpose of the present invention to provide a conveniently obtainable, inert undercoating layer on a solid phase, which layer seals small pores of the surface, which small pores are of a dimension small enough to consume binding partner, rendering it unavailable for complex formation, such as in specific binding, and which inert undercoating layer covers areas which could otherwise contribute to non-specific binding in the assay, and to which the binding partner of the assay may be conveniently coupled.

The following definitions are provided to facilitate a clear understanding of the present invention. It is noted that the use of terms in a singular tense should not be construed as to limit the applicability of the terms to use in the plural tense, i.e. binding partner or binding partners.

The term "binding partner/reactant pair" refers to any pair of molecules which exhibit mutual affinity, complexation or binding capacity, typically specific binding or interacting (complex-forming) or annealing pairs, such as antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector, complementary strands of nucleic acid, repressor/inducer, and the like.

The term "binding partner" refers to that member of a binding partner/reactant pair which is coupled to a solid phase.

The term "reactant" refers to that member of a binding partner/reactant pair, the existence and/or concentration of which is to be determined, or the recovery of which is desired, and which is to be captured by the binding partner from a medium in which it is dispersed. An analyte in a test medium is an exemplary reactant, as is a specifically-binding industrial reagent to be recovered.

The term "test assay" generally refers to any procedure in which a member of a binding partner/reactant pair in a test sample is to be detected and/or quantitated in a medium by various assay formats. For example, "test assay" may be used to describe a diagnostic procedure, analytical procedure, microanalytical procedure, forensic analysis, pharmacokinetic study, cell sorting procedure, affinity chromatogram, industrial or laboratory recovery or analysis of one or more species such as toxins, catalysts, or starting materials or products, and the like. The term may be used to describe a procedure in which a plurality of reagents, each including a different binding partner, is employed to capture a plurality of corresponding reactants. A typical test assay is an immunoassay.

The term "complex" refers to the specific binding or interaction or association of two or more species.

The term "capturing" refers to the analysis, recovery, detection, or other qualitative or quantitative determination of a reactant in a medium, for example via complexation in a test assay. As an example, in a sandwich immunoassay, a surface-coupled binding partner will form a complex with a reactant, before or after the reactant forms a complex with another (generally labeled) binding partner or reactant. In a competitive assay, labeled and unlabeled reactants typically compete for complexation with a surface-coupled binding partner.

The term "inert" refers to a chemical or biological state in which a material does not interact with the binding partner of the test assay in which it is employed in the way in which the binding partner and reactant interact. The term may also refer to a chemical or biological state in which the material does not interact with either the binding partner or the reactant in the way in which the binding partner and reactant interact.

The term "solid phase" refers to any material which is insoluble in a medium containing a reactant to be captured in a particular test assay. In a broad sense, the term describes any entity which can be substantially dispersed within a medium and removed or separated from the medium by immobilization, filtering, partitioning, centrifugation or into which the medium can be dispensed, or the like.

The term "small pores" refers to pores or other irregularities in the surface of a solid phase which are of a dimension small enough such that binding partner entering the small pores and being surface-bound therein will not be available to participate in complexation with a reactant.

The term "undercoating" refers to a layer of inert material coupled to a solid phase.

The term "intermediate" refers to a solid phase which has coupled thereto at least one undercoating layer of inert material.

The term "reagent" refers generally to a processed material modified to participate in a complexing reaction such as a test assay, more specifically to an intermediate to which a binding partner is coupled.

The term "non-specific binding" (NSB) refers to a nondesired interaction between a reagent and any other species present in a test assay other than binding partner/reactant complexation.

The term "ligand" refers to a substance or group of substances including a small molecule, such as a hapten, which is a member or a component of a binding partner/reactant pair.

The term "label" refers to an atom or a molecular moiety capable of generating a signal for detection of the binding reaction. It includes, without limitation, radioactive isotopes, enzymes, luminescent agents, precipitating agents, and dyes.

The term "sample" refers to any medium containing a reactant to be captured in a test assay.

The foregoing and other objects and advantages of the present invention are achieved by providing an intermediate comprising a solid phase having an exterior surface, an inert material covalently bound to the exterior surface to form an undercoating on the solid phase, the undercoating being selected to be amendable to couple a binding partner for capturing the reactant.

It is another object of the present invention to provide an intermediate comprising a solid phase having an exterior surface, the exterior surface coupled to a substantially non-cross-linked, inert material to form an undercoating on the exterior surface, the undercoating being selected to be amendable to mount a binding partner for capturing the reactant.

It is another object of the present invention to provide an intermediate, comprising a silanized solid phase having an exterior surface, the exterior surface coupled to an inert material forming an undercoating on the exterior surface, the undercoating being selected to be amendable to mount a binding partner for capturing the reactant.

It is another object of the present invention to provide a reagent for capturing at least one reactant with use of a binding partner of the reactant, comprising a solid phase having an exterior surface, an inert material covalently coupled to the exterior surface to form an undercoating thereon, and at least one binding partner attached to the inert material.

It is another object of the present invention to provide a reagent for capturing at least one reactant with use of a binding partner of each reactant, comprising a solid phase having an exterior surface, a substantially non-cross-linked, inert material coupled to the solid phase to form an undercoating thereon, and at least one binding partner attached to the inert material.

It is another object of the present invention to provide a reagent for binding at least one reactant with use of a binding partner of the reactant, comprising a silanized solid phase having an exterior surface, an inert material coupled to the solid phase to form an undercoating thereon, and at least one binding partner attached to the inert material.

It is another object of the present invention to provide a kit for capturing a reactant comprising a solid phase having an exterior surface, and an inert material covalently coupled to the exterior surface and forming an undercoating thereon, the undercoating being selected to mount a binding partner for capturing the reactant.

It is another object of the present invention to provide a kit for capturing a reactant, comprising a solid phase having an exterior surface, and a substantially non-cross-linked, inert material coupled to the exterior surface and forming an undercoating thereon, the undercoating being selected to mount a binding partner for capturing the reactant.

It is another object of the present invention to provide a kit for capturing a reactant, comprising a silanized solid phase having an exterior surface, and an inert material coupled to the exterior surface and forming an undercoating thereon, the undercoating being selected to mount a binding partner for capturing the reactant.

It is another object of the present invention to provide a method of making an intermediate comprising the steps of providing a solid phase having an exterior surface and covalently coupling an inert material to the exterior surface to form an undercoating thereon, the inert material selected to be suitable for mounting thereon a binding partner for capturing a reactant.

It is another object of the present invention to provide a method of capturing at least one reactant comprising the steps of providing a reagent including a solid phase having an exterior surface, the exterior surface having covalently bound thereto an inert material forming an undercoating thereon, and including at least one binding partner attached to the inert material, the binding partner being capable of forming a complex with the reactant, and forming an admixture of the reagent and a sample containing the reactant.

It is another object of the present invention to provide a method of capturing a least one reactant, comprising the steps of providing a reagent including a solid phase having an exterior surface, the exterior surface having bound thereto a substantially non-cross-linked, inert material forming an undercoating thereon, and including at least one binding partner attached to the inert material, the binding partner being capable of forming a complex with the reactant, and forming an admixture of the reagent and a sample containing the reactant.

It is still another object of the present invention to provide a method of capturing at least one reactant, comprising the steps of providing a reagent including a silanized solid phase having an exterior surface, the exterior surface having attached thereto an inert material forming an undercoating thereon, and including at least one binding partner attached to the inert material, said at least one binding partner being capable of forming a complex with the reactant, and forming an admixture of the reagent and a sample containing the reactant.

The above and other features, objects and advantages of the present invention will be better understood from the following specification when read in conjunction with the accompanying drawings in which:
Fig. 1 is a partial cross-sectional, schematic view of a portion of a surface upon which binding partner has been non-maximally loaded according to the prior art;
Fig. 2 is a partial cross-sectional, schematic view of a portion of a surface upon which binding partner has been maximally loaded according to the prior art;
Fig. 3 is a partial cross-sectional, schematic view of a portion of an idealized surface upon which binding partner and blocking agent have been loaded according to the prior art;
Fig. 4 is a partial cross-sectional, schematic view of a portion of a real surface upon which binding partner and blocking agent have been loading according to the prior art;
Fig. 5 is a partial cross-sectional, schematic view of a surface upon which undercoating and a binding partner have been loaded according to one embodiment of the present invention;
Fig. 6 is a partial cross-sectional, schematic view of a surface upon which undercoating and a binding partner have been loaded according to another embodiment of the present invention; and
Fig. 7 is a flow diagram illustrating chemical reactive pathways involved in the coupling of binding partner to solid phase, and the coupling of inert material to a solid phase to form an undercoating layer, and the coupling of binding partner to the undercoating layer

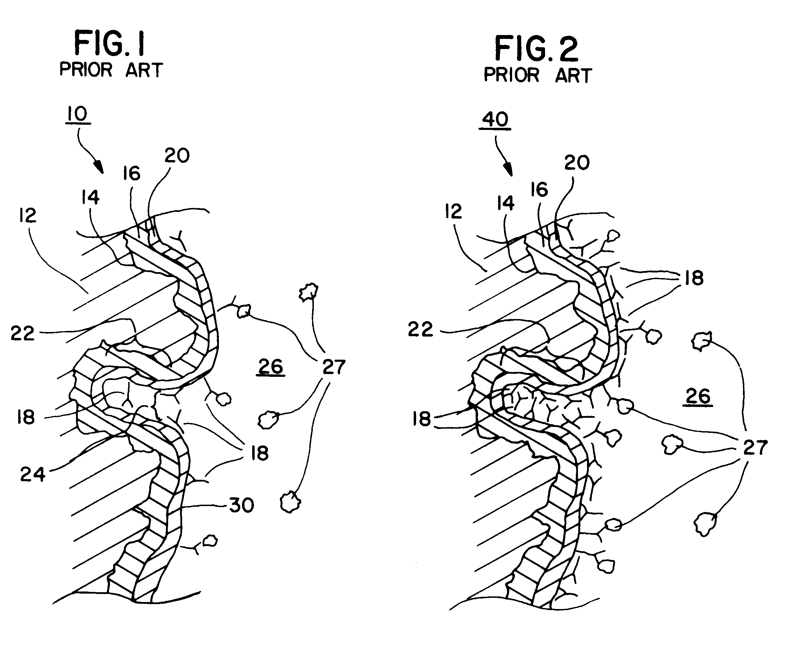

Referring to Fig. 1, a partial cross-sectional, schematic view of a magnetic particle prepared for use as an immunoassay reagent in accordance with a prior art method is illustrated generally at 10 and includes metal core 12, having a highly irregular and porous surface 14 which is coated with silane layer 16. Binding partners 18 are covalently coupled to the silanized surface via glutaraldehyde 20, which substantially completely coats silane layer 16. It is noted that Fig. 1 is for illustrative purposes only as the reaction between the silane layer and glutaraldehyde is a chemical one as is the reaction between glutaraldehyde and binding partner.

Metal core 12 typically has a highly irregular surface area, which is advantageous in many cases as a high surface area/mass ratio is desirable. Indeed, Fig. 1 could represent any area of the surface of a magnetic particle, including the interior of large irregularities or pores.

However, metal core 12 typically also includes small pores 22, into which some of the binding partners 18 locate during loading. Other binding partners may block opening 24 of small pores 22, isolating some of the binding partners within pores 22 from sample solution 26 containing reactant 27, to which prior art reagent 10 is exposed.

According to the prior art particulate reagent illustrated in Fig. 1, binding partner 18 has been loaded non-maximally. That is, an amount of binding partner less than that needed to completely coat the particle surface has been loaded, with the result that a plurality of uncovered portions 30 of the particle surface are exposed to reactant 27 in sample solution 26. Interaction of reactant 27 with uncovered portions 30 of solid phase surfaces prepared in accordance with the prior art generally leads to non-specific binding.

Binding partner 18 is directly coupled to glutaraldehyde 20 according to the prior art embodiment illustrated in Fig. 1. Glutaraldehyde coupling of binding partner to a solid phase involves poorly-understood chemistry, and includes excessive covalent binding of the binding partner, often leading to denaturization thereof.

Referring now to Fig. 2, a partial cross-sectional schematic view of a magnetic particle prepared for use as an immunoassay reagent in accordance with another prior art method is illustrated generally at 40. In Fig. 2 and in subsequent figures, references to elements common to the various figures are made using a common numerical designation for each common element. According to the method illustrated in Fig. 2 the particle is maximally loaded, that is, loaded with binding partner 18 to the extent that the surface of the particle is substantially completely covered. The particle surface is in this way "sealed", and little or no exposed particle surface area 30 exists, thus, non-specific binding may be reduced. However, a significant quantity of binding partner 18 is consumed simply to fill small pore 22, constituting waste. Additionally, to seal the surface in this manner, control of the degree of surface loading not feasible.

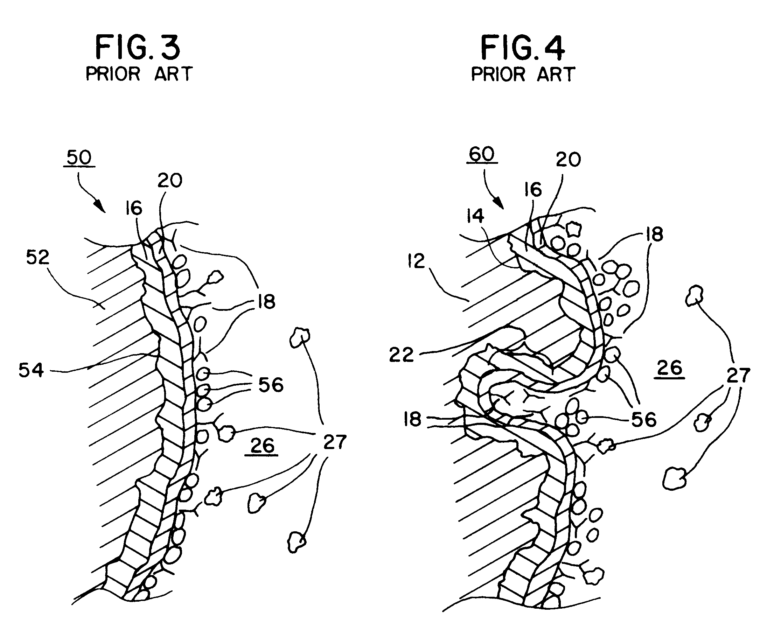

Illustrated in Fig. 3 is a partial cross-sectional, schematic view of an idealized particulate prior art immunoassay reagent 50 in which a blocking agent is employed to reduce non-specific binding while allowing less than maximal binding partner loading. Reagent 50 includes metal core 52, idealized in that no small pores exist, and having only slightly irregular surface 54. Silane layer 16 substantially completely coats surface 54 of core 52, and is activated by glutaraldehyde layer 20, to which binding partner 18 is covalently coupled. A quantity of binding partner 18 is loaded onto glutaraldehyde layer 20 in an amount less than that required to completely coat the surface of particulate prior art reagent 50. As is discussed above, this procedure is often advantageous in immunoassays, but leaves portions of the surface of the particle exposed, which contribute to non-specific binding. Therefore, after binding partner 18 is coupled to glutaraldehyde 20, blocking agent 56, typically comprising an inexpensive, immunologically inert proteinaceous material, is adsorbed onto the reagent. Blocking agent 56 ideally substantially completely covers the surface that is not coated by binding partner 18 of prior art reagent 50.

Magnetic particles generally do not have a surface free from small pores as illustrated in Fig. 3. Even if such an idealized particle existed, the idealized prior art reagent 50 illustrated in Fig. 3 includes binding partner 18 covalently linked to glutaraldehyde 20, possibly resulting in denaturization, and any blocking agent 56 that leaches out from the surface of the particle would expose surface area which could contribute to non-specific binding.

A more realistic partial cross-sectional, schematic view of a particulate prior art reagent including a blocking agent appears in Fig. 4. In Fig. 4, prior art reagent 60 is prepared according to a method including less than maximal loading of binding partner 18 followed by the application of blocking agent 56. According to the method, waste of binding partner 18 in the filling of small pore 22 exists as well as denaturization of binding partner 18 through direct coupling through glutaraldehyde 20, as well as the possibility that leaching-out of blocking agent 56 could occur, resulting in exposure of areas of the surface of the particle, leading to non-specific binding. Additionally, blocking agent 56 may cover some of binding partner 18 according to a realistic model, rendering it ineffective and constituting waste.

In all of the prior art embodiments illustrated above in Figs. 1-4, inconsistency and irreproducability of binding partner loading, non-specific binding in assay, and waste of binding partner are significant problems.

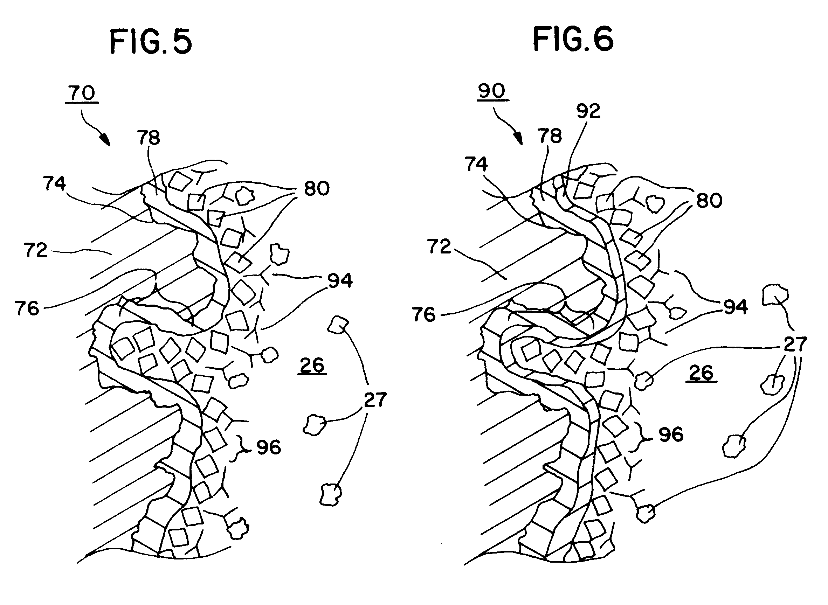

Referring now to Fig. 5 reagent 70, prepared in accordance with one embodiment of the present invention, is illustrated schematically in partial cross-section and comprises solid phase 72 having an irregular surface 74, and including coating layer 78 which may substantially completely cover surface 74.

Solid phase 72 may comprise any support conveniently employed in an immunoassay or other test assay technique, typically comprising glass plates or beads which may or may not be roughened, polymer beads such as polystyrene, silicon beads, magnetic particles such as magnetic metal particles, magnetic polyacrylamide-agarose beads, optical waveguides, test tube walls, titre plates, analyte-solution-insoluble gels such as Sephadex gel, silica, wollastonite, dried silica gel, bentonite, alumina, hydroxy apatite, and the like. According to a preferred embodiment of the present invention, solid phase 72 comprises an aggregate core of metal oxide crystals such as are described in U.S. patent no. 4,554,088, and incorporated herein by reference. The solid phase may comprise ferromagnetic metal oxide crystals, that is, those that become permanently magnetized upon exposure to a magnetic field, or superparamagnetic metal oxide crystals, that is, those that do not become permanently magnetized upon exposure to a magnetic field, superparamagnetic metal oxide crystals being preferred. Preferred superparamagnetic metal oxide crystals have a crystal size less than about 0.03 microns, and may comprise any alkaline earth or transition metal oxide material such as iron oxide, cobalt oxide, manganese oxide, magnesium oxide, nickel oxide, zinc oxide, copper oxide, or the like. The crystals form aggregate clusters preferably small enough to avoid substantial gravitational settling over times sufficient to permit dispersions of the particles to be used in a test assay or other application. Because they do not become permanently magnetized and therefore do not magnetically aggregate, the preferred particles can be redispersed.

Portions of the surface area of solid phase 72, and the entire surface area according to a preferred embodiment, are coated with a layer 78 so as to seal the metal oxide core and to provide reactive functionality for further particle modification. Preferably, layer 78 comprises a material which adheres well to core 72, and which provides exposed functional groups to which one or more types of inert materials may be coupled to form an undercoating. Such coupling may take the form of absorptive or covalent coupling, with covalent coupling being preferred. To effect covalent coupling, layer 78 preferably includes functional groups allowing covalent coupling to an undercoating layer via bonds such as amide, ester, ether, sulfonamide, disulfide, azo, or other suitable organic linkages.

According to a preferred embodiment, layer 78 comprises a silane coating. As used herein, the term silane refers to any bifunctional organosilane such as are described in U.S. patent nos. 3,652,761, 4,554,088, and 3,652,761, all of which are incorporated herein by reference. Such silanes are defined as species comprising an organo-functional and silicon-functional silicon compound characterized in that the silicon portion has an affinity for inorganic material while the organic portion is tailored to combine with organic materials. Silanes are particularly suitable for coating metal oxide cores as the silicon-functionality securely bonds to the core, and organo-functionalities are exposed for coupling to organic materials such as proteins. Preferably, the organo-functionalities of the silanes suitable for use as layers 78 in the present invention to which organic materials such as proteins may be coupled are amenable to coupling chemistries including, but not limited to, diazotization, carbodiimide, and glutaraldehyde couplings. Preferred silanes have the general formula R-Si(OX)₃, where (OX)₃ represents a trialkoxy group, typically trimethoxy or triethoxy, and R represents and aryl, alkyl or aralkyl group terminating in aminophenyl, amino, hydroxyl, sulphydryl, aliphatic, hydrophobic or mixed function (amphipathic) or other organic group suitable for coupling, preferably covalent coupling, to an undercoating layer. A non-limiting, exemplary list of suitable silanes includes p-aminophenyltrimethoxysilane, 3-aminopropyltrimethoxysilane, N-2-aminoethyl-3-aminopropyltrimethoxysilane, triaminofunctional silane n-dodecyltriethoxysilane, n-hexyltrimethoxysilane, and H₂NCH₂CH₂-NH-CH₂CH₂-NH-CH₂CH₂CH₂-Si-(OCH₃)₃.

Particularly preferred for use as coating layer 78 in the present invention is 3-aminopropyltrimethoxysilane, in which the trimethoxysilyl functionality binds securely to solid phase core 72 and which includes amino groups which are exposed at the surface of the coating 78 for coupling to organic species.

Described thus far is a preferred solid phase of the present invention, including a magnetic metal oxide core 72, coated with silane layer 78, the surface of silane layer 78 defining a preferred solid phase exterior surface of the present invention. Partially or completing coating the surface of reagent 70, the surface being defined by silane layer 78 according to preferred embodiments, and completely coating the surface according to a preferred embodiment of the present invention is an undercoating layer of inert material 80. Material 80 comprises any material which may conveniently covalently or absorptively coat the solid phase support surface. Additionally, material 80 must be inert. As used herein, the term "inert" material is meant to define a material which does not chemically or biologically interact with a binding partner, and preferably one that does not so interact with a reactant, in the way that the binding partner and complexing reactant interact. For example, in an immunoassay, material 80 must be immunologically inert with respect to the binding partner of the assay analyte, and preferably inert with respect to the analyte as well. As is apparent to one of ordinary skill in the art, the term "inert" must be defined with reference to the particular purpose for which an inventive intermediate, reagent, kit, or method is to be employed. That is, according to one embodiment, a first material may serve as inert material 80 while a second material defines a binding partner in a test assay, while in a second embodiment the second material may define the inert material 80, while the first material may define the binding partner according to that embodiment.

When inert material 80 is selected so as to covalently bind to the solid phase surface, it is selected so as to include functional groups amenable to such covalent coupling. Preferably, such functional groups are selected so as to form covalent bonds which may be amide, ester, ether, sulfonamide, disulfide, azo, and the like. Thus, material 80 advantageously comprises material including at least one type of functional group selected from the group consisting of -S-S, -NR₂, -COOR, -SR, -COR, -OR, and -OCOR, where R is hydrogen or an organic group such as a hydrocarbon. As used herein, the term "hydrocarbon" includes alkyl, alkenyl, alkynyl, cycloalkyl, aryl, alkaryl, aralkyl, and the like. The hydrocarbon group may, for example, comprise methyl, propenyl, ethynyl, cyclohexyl, phenyl or derivative, tolyl, and benzyl groups.

Inert material 80 may comprise a proteinaceous or non-proteinaceous material, preferably a proteinaceous material which may be relatively easily obtained in sufficient quantity to completely coat the surface of a relatively large quantity of solid phase material, including the filling of surface irregularities such as small pores 76. Additionally, inert material 80 may be advantageously selected so as to maintain its structural integrity over a relatively long shelf life of solid phase so coated to form an intermediate or, when further processed, a reagent.

A non-limiting exemplary list of materials suitable for use as inert material 80 according to the present invention includes proteinaceous material such as bovine serum albumen (BSA), bovine gammaglobulin (BGG), apoferritin, ovalbumin, avidin, streptavidin (SAV), biotin, peptides, polyamino acids, synthetic polyamino compounds, and non-proteinaceous polyamino acids.
Particularly preferred are bovine serum albumin and bovine gamma globulin.

A variety of coupling chemistries may be employed to covalently couple inert material 80 to a solid phase surface, the particular chemistry selected being somewhat dependent upon the functional groups that are exposed at the surface of layer 78 and functional groups included in material 80 and available for coupling. According to a preferred embodiment in which layer 78 comprises silane and includes exposed -NH₂ groups available for coupling, material 80 comprises a proteinaceous material including carboxyl groups available for bonding, such as bovine gamma globulin. According to this embodiment an agent selected so as to activate the carboxyl groups may be employed, excess amounts of such reagent being advantageously relatively easily washed away after coupling is completed. A non-limiting exemplary list of such activating agents includes benzotriazolyl-N-oxy-tris(dimethylamino)phosphonium hexafluorophosphate; o-benzotriazol-1-yl-N,N,N',N'-tetramethyluronium hexafluorophosphate; o-benzotriazol-1-yl-N,N,N',N'-tetramethyluronium tetrafluoroborate; 1, 1'-carbonyldiimidazole; 1-cyclohexyl-3-(2-morpholinoethyl)carbodiimide metho-p-toluenesulfonate; N,N'-dicyclohexylcarbodiimide; N,N'-dicyclohexylcarbodiimide pentachlorophenol complex; N,N'-diisopropyl carbodiimide; 5,5'-dimethyl-1,3-cyclohexanedione; diphenylphosphinyl chloride; diphenylphosphoryl azide; diphenylphosphoryl chloride; 2,2'-dithiodipyridine; N-ethoxycarbonyl-2-ethoxy- 1 ,2-dihydroquinoline; 1-ethyl-3-(3-dimethylaminopropyl) carbodiimide; 1-ethyl-3-(3-dimethylaminopropyl)carbodiimide methiodide; N-ethyl-5-phenylisoxazolium-3'-sulfonate; N-hydroxy-5-norbornene-2,3-dicarboximide; isobutyl chloroformate; and bis(2-oxo-3-oxazolidinyl)-phosphonic chloride.
A particularly preferred agent is 1-ethyl-3-(3-dimethylaminopropyl)carbodiimide hydrochloride (EDC). According to this embodiment, inert material 80 is directly covalently coupled to the exterior surface of the solid phase.

Also within the scope of the present invention are included methods of covalently coupling inert material 80 to layer 78 using homobifunctional or heterobifunctional linking agents. Illustrative of this embodiment and with reference to Fig. 6, reagent 90 is shown in partial cross-section schematically and includes linking agent 92 covalently coupled to layer 78 and to which inert material 80 is covalently coupled. Linking agent 92 may comprise any of a variety of bifunctional agents which have functional groups amenable to covalent coupling with the above-described functional groups which may exist on the exposed surface of layer 78, and amenable to covalent coupling with the above-described functional groups which may be available for bonding on inert material 80, the respective coupling chemistry resulting in covalent bonds between layer 78 and linking agent 92 and between linking agent 92 and inert material 80 such as amide, ester, ether sulfonamide, disulfide, azo, or other organic linkage. A non-limiting exemplary list of heterobifunctional linking agents suitable for use as linking agent 92 in the present invention includes: Succinimidyl 4-(N-maleimidomethyl)cyclohexane-1-carboxylate, Sulfosuccinimidyl 4-(N-maleimidomethyl)cyclohexane-1-carboxylate, m-Maleimidobenzoyl-N-hydroxysuccinimide ester, m-Maleimidobenzoyl-N-hydroxysulfosccinimide ester, N-succinimidyl-3-(2-pyridyldithio)propionate, Succinimidyl 4-(p-maleimidophenyl)butyrate, Sulfosuccinimidyl 4-(p-maleimidophenyl)butyrate, N-Succinimidyl(4-iodoacetyl)aminobenzoate, Sulfosuccinimidyl(4-iodoacetyl)aminobenzoate, 1-Ethyl-3-(3-dimethylaminopropyl)carbodiimide Hydrochloride, and N-Hydroxysulfosuccinimide. A non-limiting exemplary list of homobifunctional linking agents suitable for use as agent 92 of the present invention includes: Ethylene glycolbis(succinimidylsuccinate), Ethylene glycolbis(sulfosuccinimidylsuccinate), Disuccinimidyl tartarate, Disulfosuccinimidyl tartarate, Bis[2-(succinimidooxycarbonyloxy)ethyl]sulfone, Bis[2-(sulfosuccinimidooxycarbonyloxy)ethyl]sulfone, Dimethyl adipimidate.2HCl, Dimethylpimelimidate.2HCl, Dimethylsuberimidate.2HCl, Dimethyl-3,3'-dithiobispropionimidate.2HCl,Bismaleimidohexand,,5-Difluoro-2,4-dinitrobenzene, 4,4'-Diisothiocyano-2,2'disulfonic acid stilbene disodium salt, Disuccinimidyl suberate, Bis(sulfosuccinimidyl)suberate, Dithiobis(succinimidylpropionate), and 3,3'-Dithiobis(sulfosuccinimidylpropionate). Additionally, glutaraldehyde may be used as agent 92 for coupling undercoating 80 to layer 78, and this is preferred particularly when layer 78 comprises a silane coating and undercoating 80 comprises bovine serum albumin.

As discussed above, according to certain embodiments of the present invention inert material 80 is absorptively coated onto the surface of the solid phase. Such absorptive coating of the solid phase may be especially effective when the solid phase has an especially irregular surface or in other situations in which van der Waals interactions between inert material 80 and the surface of the solid phase would be maximized. Such absorptive coating may be especially effective when the solid phase comprises un-silanized or silanized magnetic metal particles, or roughened surfaces such as roughened glass beads, roughened polymer beads, roughened plates or the like. When such absorptive coupling of inert material 80 to the solid phase surface is exploited, inert material 80 may be intermolecularly cross-linked or may remain substantially non-cross-linked. According to a preferred embodiment, inert material 80 remains substantially non-cross-linked when absorptively coupled to the solid phase. When absorptive coupling of inert material 80 to the solid phase is chosen, the stability of the absorptive interaction may be tested by determining the degree of non-specific binding of a reagent including an undercoating layer of inert material 80 in a capturing process such as an immunoassay.

As described thus far, inert material 80 of the present invention is absorptively or covalently coupled to the surface of a solid phase to be used in a test assay such as an immunoassay or the like, the material being selected so as to be inert with respect to a binding partner, and preferably with respect to a reactant such as an analyte that forms a complex with the binding partner. Material 80 may also be advantageously selected so as to be easily obtainable, such that the solid phase surface may be relatively inexpensively completely coated so as to fill small pores 76 and to assure complete surface coverage so as to minimize non-specific binding in an assay. Inert material 80 advantageously coats the solid phase surface to the extent that small pores 76 are filled or otherwise blocked and the surface is substantially completely covered, but is not loaded onto the surface to the extent that the amount of surface area available for binding partner coupling is reduced. This results from the step described below in the Examples in which excess binding partner is washed from the surface after coupling.

The invention as described thus far comprises a solid phase, the exterior surface of which is covered, and covered substantially completely according to preferred embodiments, with inert material 80 absorptively or, preferably, covalently, to form an undercoating. This article may serve as an intermediate product or precursor for making the reagent according to the second aspect of the invention described below. The intermediate may be prepared and stored, and used as a basis for formation of a variety of reagents for use in a variety of test assays.

A particular advantage of preparation of such an intermediate including the inventive inert material is in the storage of these materials for subsequent preparation of reagents. When conventional silanized magnetic particles are stored in intimate interparticle contact, interparticle bonding, for example through cross-linking via siloxane formation, may yield aggregates of silanized particles of considerably larger diameter than individual particles. Such aggregates have low surface area per unit weight, hence a low capacity for coupling with binding partners. Such aggregates also have gravitational settling times which are too short for many applications. The coated particles of the present invention alleviate this interparticle aggregation problem.

According to a second aspect of the invention, and with reference to Figs. 5 and 6, binding partner 94 is coupled to inert material 80, to form a reagent for use in a test assay, so as to be advantageously exposed to solution 26 including reactant 27. Binding partner 94 may be absorptively or covalently coupled to inert material 80, and is covalently coupled thereto in a preferred embodiment.

Binding partner 94 may be selected so as to capture a wide variety of reactants including hormones, pharmacologic agents, vitamins, cofactors, hematological substances, virus antigens, nucleic acids, nucleotides, glycosides, sugars, toxins, ligands, nucleic acid strands, target molecules, and the like. Thus, binding partner 94 may be selected so as to allow the reagent, kit, and/or method of the present invention to be utilized in diagnostic procedures, microanalytical procedures, forensic analyses, pharmacokinetic studies, cell sorting systems, affinity chromatography, industrial or laboratory recovery or analysis of species such as toxins, catalysts, or starting materials or products, and the like. Applicable cell sorting systems are described in U.S. patent nos. 3,970,518, 4,230,685, and 4,267,234, all of which are incorporated herein by reference. Applicable affinity chromatography is described in an article by K. Mosbach and L. Andersen in Nature, vol. 170, pp. 259-261 (1977), incorporated herein by reference. Additional interactions which may be exploited by the reagent of the present invention, and from which selection of binding partner 94 may be made, are included in U.S. patent no. 4,554,088, referenced above.

Preferably, binding partner/reactant pairs according to the invention comprise interacting pairs such as antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector, repressor/inducer, or the like. Thus, binding partner 94 may be selected from the group consisting of antibody, antigen, hapten, enzyme, enzyme substrate, nucleic acid sequence, inhibitor, cofactor, binding protein, binding protein substrate, carrier protein, carrier protein substrate, lectin, carbohydrate, hormone, hormone receptor, hormone effector, hormone repressor, hormone inducer, and the like. In a particularly preferred embodiment, binding partner 94 is an antibody or antigen.

Of the above-noted exemplary binding partner/reactant pairs, designation as the binding partner and designation as reactant will be defined by which species is to be captured from solution and which is to be bound to a solid phase. The member to be captured from solution is termed the reactant, and the member coupled to the solid phase is termed the binding partner. As would be apparent to one skilled in the art, one species could serve as a binding partner in one test assay, and a reactant in another.

Binding partner 94 preferably includes an exposed functional group amenable for covalent bonding to inert material 80, such as those functional groups described above with respect to preferred materials suitable for use as material 80. According to the chemistries defined herein with respect to covalent coupling of material 80 to layer 78, either directly as described with respect to Fig. 5 or through linking agent 92 as described with respect to Fig. 6, particular reactive groups of material 80 may be exposed, that is, may be oriented so as to be amenable for complexing with a reactant. For example, when material 80 is covalently coupled to layer 78 via glutaraldehyde as linking agent 92, -NH₂ groups of material 80 are substantially consumed in the reaction, and multiple carboxyl groups remain exposed. Thus, an activating agent which may activate carboxyl groups on material 80 for further coupling to -NH₂ groups of binding partner 94 may be employed, such as EDC. When a binding partner 94 including, for example, exposed aldehyde groups is employed, reductive coupling using a reagent such as sodium cyanoborohydride may be used. Alternatively, heterobifunctional linking agents such as those described above may be employed.

Preferably, any mode of coupling of binding partner 94 to material 80 should be selected so as not to adversely affect binding partner 94. In order to select the optimum coupling agent or agents, it is important to consider the active sites and the susceptibility of the binding partner to denaturization. This applies especially to antibodies. A coupling agent should be selected which is nondestructive to the antibody, as for example, by bonding to the tyrosine or sulfhydryl group of the antibody. Furthermore, the coupling agent must be such that bonding can be produced under conditions (for example, temperature and pH) that do not destroy either the binding partner or the solid phase support surface.

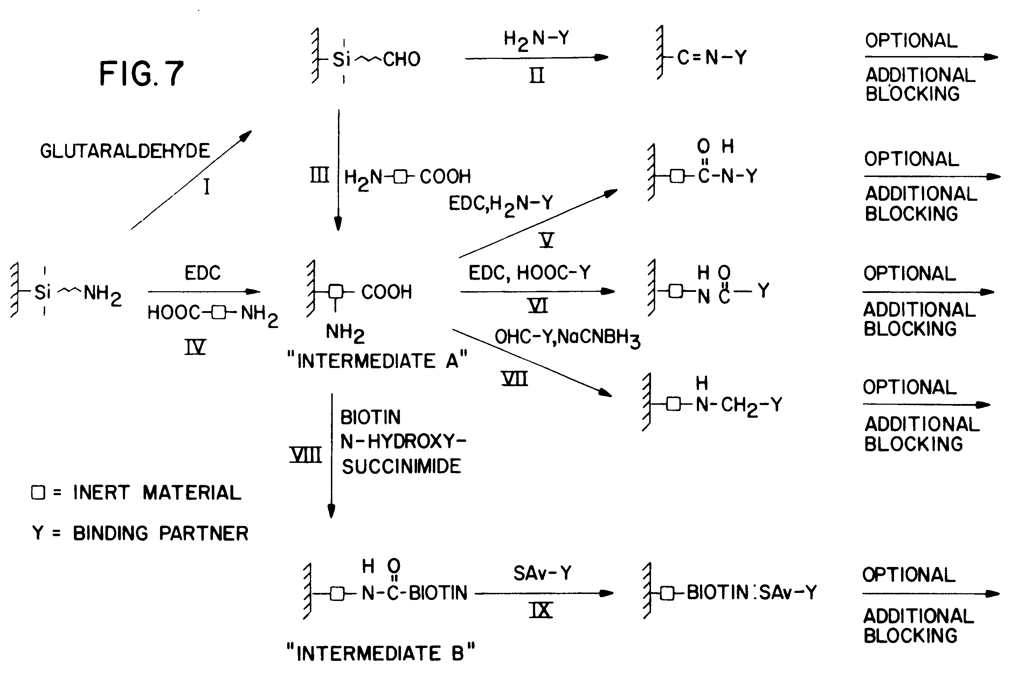

The reagent may be additionally blocked prior to use in test assays. A preferred material for the second coating is an inert material and, particularly, BSA or BGG. See Fig. 7 optional additional blocking.

In Fig. 7, a flow chart illustrating exemplary chemical pathways for the coupling of binding partner to a solid phase according to the prior art, and according to exemplary embodiments of the present invention, is illustrated.

With reference to reaction I, a silanized surface having exposed amine groups is activated with glutaraldehyde, resulting in a network including exposed aldehyde functionalities. According to the prior art, and with reference to reaction II, a binding partner having an amine functionality is then coupled thereto. According to the present invention, however, and with reference to reaction III, an inert material 80 for forming an undercoating layer and comprising both amine and carboxyl functionalities may be coupled to glutaraldehyde-functionalized solid phase, resulting in "intermediate A".

Alternatively, direct covalent coupling of an inert material to form an undercoating layer on the solid phase, that is, covalent coupling without a cross-linking agent such as glutaraldehyde, is illustrated with reference to reaction IV. According to the reaction, an inert material for forming an undercoating layer, the material including a carboxyl functionality and an amine functionality, is activated with an activating agent such as EDC, and allowed to react with exposed amine groups of the silane layer to form "intermediate A".

Subsequently, at least two general strategic pathways exist for the coupling of a binding partner to the resultant intermediate, one involving a variety of chemical pathways and one involving specific binding. The chemical pathways will be described first. According to reaction V, carboxyl functionalities of the undercoating layer are activated with an agent such as EDC, followed by reaction of the activated carboxyl groups with amine groups of binding partner. According to reaction VI, carboxyl groups of the binding partner may be activated by EDC, followed by coupling to amine groups on the undercoating layer. According to reaction VII, aldehyde groups on a binding partner may be coupled to the amine functionality of the undercoating layer, followed by a reductive reaction with a reducing agent such as NaCNBH₃. According to a pathway involving specific binding, and with reference to reaction VIII, an indirect immobilization of a binding partner onto the intermediate may be effected. With reference to reaction VIII, biotin derivative having a functional group which may covalently couple to an exposed functional group of the undercoating layer, for example biotin N-hydroxysuccinimide may be covalently coupled to the undercoating layer to form "intermediate B", followed by coupling of an avidin or streptavidin-modified binding partner to the exposed, undercoating-layer-bound biotin (reaction IX). Alternately, an avidin or streptavidin species may be covalently coupled to the undercoating layer, followed by introduction of a biotin-bound binding partner.

Of course, the flow chart of Fig. 7 provides exemplary pathways only. As would be apparent to one of ordinary skill in the art, a variety of pathways for coupling of inert material to a solid phase to form an intermediate including an inert undercoating layer, and the coupling of binding partner to the undercoating layer are available, and are understood to be within the scope of the present invention. It is to be understood that the concept of the present invention resides in the provision of an inert material undercoating layer on the exterior surface of solid phase materials, upon which binding partner material may be coupled, and that the particular chemical pathways exploited for the coupling steps described may be widely varied so long as significant denaturization of binding partner 94 does not result.

According to the present invention, binding partner may be loaded onto a solid phase to produce reagents 70 or 90 in a predetermined amount so as to tailor the reactivity of the reagent. The reactivity may thus be relatively predictable based upon the amount of binding partner 94 added, as binding partner 94 is substantially exclusively located at exposed sites of reagents 70 or 90 accessible to reactants 27 in solution 26, and is not consumed in the filling of small pores 76. The amount of binding partner 94 added is generally advantageously less than that needed to completely cover the surface area of reagents 70 or 90. According to the invention, sites 96 of the inventive reagents which remain uncovered by binding partner 94 do not add to non-specific binding of an assay since they merely comprise inert material 80, rather than, for example, glutaraldehyde layer 92 or silane layer 78. A further blocking step may optionally be performed prior to the reagent's use in a test assay.

Also provided in accordance with the present invention is a kit for capturing a reactant in a medium, or recovering a particular toxin, catalyst, industrially-important or expensive starting material or reactant from a medium. According to this embodiment of the invention, the kit includes a solid phase, inert material coupled to the solid phase surface to form an undercoating layer, preferably covalently coupled, and the kit may also include a binding partner attached to the inert material undercoating layer.

According to any of the embodiments of the present invention, the kit advantageously further comprises a reactor for reacting the reagent with the reactant (and further reaction where appropriate, as in a forward sandwich assay). Such a reactor may take a wide variety of forms, for example a simple test tube, a stirred or otherwise agitated solution, a column through which fluid containing a reactant is passed, or the like. Additionally, the kit advantageously includes an isolator for isolating the reagent and all materials complexed thereto. The isolator may be a permanent magnet, an electromagnet, or other conventional means for generating a magnetic field. Alternatively, the isolator may be a simple physical separating means such as a centrifuge, a sieve, or other filtering means.

Additionally, the kit advantageously comprises a detector for detecting the presence of, or relative quantity of, label attached to the solid phase. Such a detector may be any conventional detector suitable for the particular label used, for example a radioactivity measuring device, a visual means such as microscope or the naked eye, a spectrometer or spectrophotometer, or the like. Alternatively, the detector may comprise a chemical reagent which reacts with the solid phase, signalling the presence of and/or relatively quantity of label. Such chemical detecting means may be used in concert with other detecting means.

The following examples are intended to illustrate the benefits of the present invention, but do no exemplify the full scope of the invention. For example, although magnetic particles are exclusively exemplified, as described above a variety of solid phases including glass or polymer beads, titre plates, test tubes, Sephadex gel or the like may be employed. Although glutaraldehyde and EDC coupling of undercoating are exclusively exemplified in attachment of the undercoating to the solid phase support, a variety of alternate chemistries may be employed. Additionally, a variety of support surfaces may be modified with the undercoating of the present invention, including surfaces used in cell sorting systems, affinity chromatography systems, in vivo systems for diagnostic localization of cells or tissues recognized by a particular binding partner, directed delivery of therapeutic agents coupled to surfaces, and the like.

It is to be understood that the invention resides in the introduction of an undercoating layer to a solid phase surface, onto which a binding partner is coupled, and the above-described and other modifications and their equivalents are understood to be within the scope of the present invention.

### EXAMPLE 1

### Coupling of Undercoating Layer to Magnetic Particles to Form an Intermediate

A magnetic particle solid phase in which an undercoating layer is covalently coupled to the particles through the bifunctional cross-linking agent glutaraldehyde, to form an intermediate which can then be further processed to form a reagent, is described. Silanized superparamagnetic particles, specifically, aggregates of iron oxide crystals coated with N-2-aminoethyl-3-aminopropyltrimethoxysilane, were purchased from Advanced Magnetics, Inc. of Cambridge, Massachusetts. Horse apoferritin and glutaraldehyde were purchased from Sigma Chemical Co., St. Louis, Missouri.

150 mg of silanized particles were washed 3 times with 5 ml of methanol with magnetic separations between washings, then 3 times with 5 ml of 10mM phosphate buffer, 0.02% NaN₃, pH 7.4 (wash buffer). The magnetically separated material or wetcake was resuspended in 3 ml of wash buffer and added with 2.5 ml of a 5% glutaraldehyde solution in wash buffer. After mixing by shaking for two hours at room temperature (RT), the glutaraldehyde activated particles were washed three times with 6 ml of wash buffer. 50 mg of these particles were resuspended in 1.5 ml of wash buffer and added with a solution of 5 mg of horse apoferritin in 0.1 ml of 0.15M NaCl, and the suspension was mixed for two hours at RT. The apoferritin-coated particles were then washed 3 times with 2 ml of wash buffer, then resuspended in 2 ml of wash buffer and stored at 4°C until use. These intermediate magnetic particles having a horse apoferritin undercoating can be further processed to form a reagent for use in a test assay.

### EXAMPLE 2

### Coupling of Anti-hCG Antibody to Intermediate

Magnetic particulate intermediate from Example 1 was processed to form a reagent for use in an hCG assay. Monoclonal anti-human chorionic gonadotropin (anti-hCG) antibody, Lot #AS11, was from ACS™ Total hCG Assay, Ciba Corning Diagnostics Corp., Medfield, Massachusetts, and was utilized as a binding partner. 1-Ethyl-3-(3-dimethylaminopropyl)carbodiimidehydrochloride (EDC) was purchased from Sigma. 50 mg of magnetic particulate intermediate from Example 1 was resuspended in 1.5 ml of 20 mM 2-[N-morpholino]ethanesulfonic acid (MES), pH 5.5. A solution of 0.1 g of EDC in 0.2 ml of 20mM MES was then added and the suspension was mixed again for 30 minutes at RT. A solution of 8.0 mg of anti-hCG antibody in 1.5 ml of 50 mM phosphate buffer, pH 8.0, was added and the particles were mixed for another 16 hours at RT. The resulting antibody-coated particulate reagent was washed with 2 ml of wash buffer, 2 ml of 1M NaCl, and three times with 10 mM phosphate buffer containing 0.5% bovine serum albumin (BSA), pH 7.4 (PBS-BSA).

After washing, the particulate reagent was resuspended and blocked in 2 ml of PBS-BSA and incubated at 50°C overnight. This additional blocking step at 50°C provided a reagent with reduced non-specific binding characteristics as shown by Example 3. The particles were then washed 3 times with 2 ml of wash buffer, resuspended in 2 ml of PBS-BSA, and stored at 40C until use.

Alternatively, the reagent may be utilized without the performance of the additional blocking step.

### EXAMPLE 3

### Total hCG/Immunoassay

The additionally blocked reagent prepared in Example 2 was used in a sandwich assay to demonstrate its clinical utility. hCG serum standards and Lite Reagent, a polyclonal anti-hCG antibody labeled with acridinium ester, were all obtained from the above ACS™ Total hCG Assay. The assay procedure was as follows: 50 ul of hCG standard was allowed to mix with 100 ul of Lite Reagent and 450 ul of the reagent suspension of Example 2. The mixture was incubated at 37°C for 7.5 minutes. The particles were magnetically separated, washed with 1 ml of deionized water (DI water), and resuspended in 100 ul of DI water. The chemiluminescent signal in Relative Light Units (RLU) was read on a Magic Lite Analyzer MLA-1, Ciba Corning Diagnostics Corp. as shown in Table 1.

**Table 1**

| hCG Concentration | RLU (%CV) | %B/Bmax |
|---|---|---|
| 0 mIU/ml | 3,043 (5.0%) | 0.4% |
| 5 | 11,170 (2.7%) | 1.6 |
| 25 | 40,420 (5.3%) | 5.7 |
| 100 | 148,100 (2.7%) | 20.7 |
| 500 | 460,950 (4.2%) | 64.4 |
| 1200 | 715,360 (2.4%) | 100. |

A dose response curve for hCG standards was drawn by plotting %B/Bmax vs. doses, where B is the signal of the bound standard and Bmax is the signal of the bound 1200 mIU/ml standard. The results demonstrate that a sensitive hCG assay can be achieved by using a magnetic particulate reagent prepared according to the present invention. The high binding capacity of this solid phase also allows the assay to show no "hook effect" even at a concentration of 1 million MIU/ml of hCG, with the bound signal being 212% of Bmax.

### EXAMPLE 4

### Direct Coupling of Undercoating Layer to Magnetic Particles to Form an Intermediate

A magnetic particle solid phase in which an undercoating layer is directly covalently coupled to the particles without a bifunctional cross-linking agent is described. 413 mg of silanized particles were washed 3 times with 16 ml of methanol, then 3 times with 16 ml of wash buffer, and the wetcake resuspended in 4 ml of wash buffer. 300 mg of EDC in 1 ml of DI water was then added, immediately followed by the addition of 200 mg of BSA in 8 ml of wash buffer, and the suspension mixed for 3 hours at RT. The BSA-coated particulate intermediate was processed similarly to that of Example 1 and stored at 4°C until use. These intermediate magnetic particles can be further processed to form a reagent for use in a test assay.

### EXAMPLE 5

### Coupling of Digitoxin to Intermediate

Based on the theory of hapten heterology as described in U. Piran et al, J. Immuno. Methods, vol. 133, 207 (1990), magnetic particulate intermediate from Example 4 was processed to form a reagent carrying digitoxin as binding partner for use in a digoxin assay. 180 mg of particles were resuspended in 8 ml of 50 mM carbonate buffer, pH 9.5. Following a procedure by T. Smith et al., Biochem., vol. 9, 331 (1970), 180 mg of digitoxin dialdehyde was freshly prepared and added to the particles. The suspension was mixed for 2 hours. 150 mg of sodium cyanoborohydride in 3 ml of DI water was then added and the suspension was further mixed for 16 hours. The digitoxin-coated particles were washed with 2 ml of wash buffer, 2 ml of 1M NaCl, and three times with PBS-BSA.

After washing, the particulate reagent was resuspended in 2 ml of PBS-BSA and incubated at 50°C overnight as an additional blocking step, then washed 3 times with wash buffer, resuspended in 2 ml of PBS-BSA, and stored at 4°C until use.

Alternatively, the reagent may be utilized without the performance of the additional blocking step.

### EXAMPLE 6

### Digoxin Assay

The additionally blocked digitoxin-coated particulate reagent prepared according to Example 5 was evaluated in a digoxin assay using serum digoxin standards and a chemiluminescent Lite Reagent, a monoclonal anti-digoxin antibody labeled with acridinium ester (MAb-AE), all from ACS™ Digoxin assay, Ciba Corning Diagnostic Corp., with the following assay protocol. 100 ul digoxin standard was added to 50 ul of Lite Reagent (total counts: 3.3 million RLU). The mixture was incubated at 370C for 2.5 minutes. 500 ul of digitoxin-coated particulate reagent of Example 5 at 100 ug/ml concentration was then added, and the mixture was incubated at 37°C for another 5 minutes. Particles were separated, washed twice with 1 ml of DI water, and resuspended in 100 ul of DI water. The assay signal was read on the MLA-1 and the results are outlined in Table 2.

**Table 2**

| Digoxin | RLU (% CV) | %B/Bo |
|---|---|---|
| 0 | 108,850 (1.0%) | 100 % |
| 0.5 ng/ml | 77,603 (3.2%) | 71.3% |
| 1.0 | 62,393 (5.8%) | 57.3% |
| 2.0 | 47,407 (3.8%) | 43.5% |
| 4.0 | 35,823 (3.2%) | 32.9% |
| 6.0 | 30,800 (8.9%) | 28.3% |

A dose response curve for the digoxin standard was drawn by plotting %B/Bo vs. doses, where B is the signal of the bound standard and Bo is the bound signal at zero dose. A sensitive digoxin assay was demonstrated with assay sensitivity estimated at less than 0.1 ng/ml and the ED50 estimated at 1.56 ng/ml. The effectiveness of the direct coupling of an undercoating layer to the silanized particle surface and the application of the present invention in the conjugation of small molecular-weight compounds to the undercoating layer to form a reagent for use in a test assay are therefore confirmed.

### EXAMPLE 7

### Comparison with Prior Art Processes

Silanized magnetic particles were coated with a binding partner, specifically an affinity purified goat anti-mouse antibody (GAMAb) obtained from Magic® Lite T-UP assay, Ciba Corning Diagnostics Corp. Both a conventional procedure as described in U.S. patent no. 4,554,088, and a procedure according to the present invention, a modification of Example 1, using BSA as a proteinaceous undercoating and glutaraldehyde as a cross-linking agent were used in this study. In each procedure, three different loadings of GAMAb at 8, 4, 0.8 mg were coupled respectively onto three batches of 50 mg particles, followed by the additional blocking of Example 2.

In order to compare the binding capacities and NSB characteristics of these reagents, all six batches of the GAMAb-coated particles were allowed to react with two Lite Reagents: monoclonal anti-pyridinoline antibody labeled with acridinium ester (MAb-AE, total counts: 2.8 million RLU), and GAMAb labeled with acridinium ester (GAMAb-AE, total counts: 2.58 million RLU). The MAb-AE will bind specifically to GAMAb on the magnetic particles while any reaction of GAMAb-AE on the particles is considered NSB. 100 ul of either Lite Reagent was allowed to mix with 500 ul of GAMAb-coated particles at 100 ug/ml concentration and the mixture incubated at 37°C for 7.5 minutes. The particles were separated magnetically, washed twice with 1 ml of DI water, and resuspended in 100 ul of DI water. The chemiluminescent signal was read on the MLA-1 analyzer.

The assay results of using the particles prepared according to a conventional procedure are outlined in Table 3.

**Table 3**

| GAMAb Loading | Signal (%CV) | NSB (%CV) | Signal/NSB Ratio |
|---|---|---|---|
| 8 mg | 796,120 RLU (1.2%) | 12,597 RLU (2.9%) | 63.2 |
| 4 mg | 887,653 (1.9%) | 37,663 (1.5%) | 23.6 |
| 0.8 mg | 819,843 (1.0%) | 74,930 (0.5%) | 10.9 |

It is clear that as the antibody loading decreases, the amount of NSB increases dramatically because of increased exposure of unprotected surface. In order to suppress NSB, one normally needs to use a large excess of binding partner in the immobilization procedure.

The reagents prepared according to the present invention were tested using the same protocol as mentioned above. The results are summarized in Table 4.

**Table 4**

| GAMAb Loading | Signal (%CV) | NSB (%CV) | Signal/NSB Ratio |
|---|---|---|---|
| 8 mg | 811,353 RLU (0.9%) | 9,973 RLU (1.9%) | 81.4 |
| 4 mg | 647,027 (6.2%) | 10,627 (5.8%) | 60.9 |
| 0.8 mg | 319,233 (1.0%) | 12,513 (10.3%) | 25.5 |

Reagents prepared according to the present invention showed smaller magnitudes of NSB than those prepared according to the conventional procedure because of the effective coverage of solid phase surface with the undercoating layer. For the same reason, little increase of NSB was seen as the antibody loading decreases. It is noteworthy that when 8 mg of antibody was used in the new coating process, the assay signal was approximately the same as the optimized result from the conventional process. Also, with reagent prepared according to the present invention the assay signal is observed to be proportional to the amount of antibody binding partner used in the coupling procedure. This suggests that the number of binding sites on the particle surface can be tailored to meet any specific assay's need. Comparison of signal/NSB ratios from Tables 3 and 4 further reveals that significantly better ratios have been achieved using procedures according to the present invention at different concentrations of antibody loading. Specifically, the signal/NSB ratio of the 0.8 mg-loaded reagent prepared according to the present invention is very close to that of the 4 mg-loaded reagent prepared according to the prior art, thus a five-fold increase in loading is required using the prior art method to achieve the same signal/NSB as that realized according to the present invention in that range of loading.

This experiment may be similarly conducted with any commercially-available monoclonal antibody.

### EXAMPLE 8

### Coupling of Undercoating Layer to Magnetic Particles

Using a procedure similar to that of Example 1, one gram of silanized particles were coated with 160 mg of bovine gammaglobulin undercoating (BGG, from ICN, Irvine, CA, Lot #B005) to form an intermediate. The intermediate was processed and stored as described in Example 1.

### EXAMPLE 9

### Coupling of Biotin to Intermediate

The intermediate from Example 8 was processed to form a modified intermediate. The BGG-coated particles were resuspended in 16 ml of wash buffer followed by addition of a solution of 60 mg biotin amidocaproate N-hydroxysuccinimide (from Sigma, B-2643) in 3 ml of N,N-Dimethylformamide. The suspension was mixed for 3 hours at RT and the resulting biotin-coated modified intermediate was additional blocked as described in Example 2. The modified intermediate may be utilized as a precursor for preparing alternate types of reagents utilizing an avidin or streptavidin labelled binding partner.

Alternatively, the modified intermediate may be utilized without the performance of the additional blocking step.

### EXAMPLE 10

### Coupling of Assay Binding Partner to Biotin Modified Intermediate

To effect an indirect immobilization of a binding partner (antibody or antigen) onto the reagent of the present invention, the biotinylated modified intermediate from Example 9 can be used along with an avidin or a streptavidin conjugated to the antibody or antigen to be immobilized, to form an immunoassay reagent. A streptavidin-PYD conjugate was obtained from PYRILINKS Assay, Metra Biosystems, Inc. of Palo Alto, California. 25 ul of streptavidin-PYD at 1 ug/ml concentration and 200 ul of biotin-coated modified intermediate (Example 9) at 200 ug/ml concentration were mixed, processed, and stored as described above with respect to Examples 1 and 2.

### EXAMPLE 11

### Pyridinoline Assay

A competitive immunoassay measuring pyridinoline (PYD), a bone resorption marker as reported in S. Seyedin et al., J. Bone & Mineral Res., vol. 8, 635 (1993), is developed to illustrate the advantageous utility of the indirect immobilization of a binding partner in Example 10.

PYD standards were obtained from PYRILINKS Assay, Metra Biosystems, Inc. Monoclonal anti-PYD antibody lot #3G6 was also from Metra Biosystems and was labeled with acridinium ester, and the resulting MAb-AE was used as Lite Reagent in the assay conducted with the following protocol: 200 ul of PYD standard was reacted with 25 ul of Lite Reagent (total counts: 3.8 million RLU) and the pre-mixed solid phase reagent of Example 10. The mixture was incubated at 37°C for 7.5 minutes and the particles were separated, washed with 1 ml of DI water, and resuspended in 100 ul of DI water. The data from the MLA-1 are outlined in Table 5.

**Table 5**

| Pyridinoline | RLU (% CV) | %B/Bo |
|---|---|---|
| 0 | 521,767 (4.2%) | 100 % |
| 50 nM | 440,913 (2.4%) | 84.5% |
| 150 | 359,840 (0.8%) | 69.0% |
| 300 | 276,903 (3.4%) | 53.1% |
| 600 | 195,547 (7.7%) | 37.5% |

A sensitive pyridinoline assay standard curve was generated to demonstrate the use of a streptavidin-biotin binding system in conjunction with the present invention. This approach will allow for a biotin-coated reagent to be mixed with an avidin conjugated to a binding partner to form a further reagent, and the resultant reagent can be used directly in a particular assay.

## Claims

1. A reactant capture intermediate characterised in that it comprises:
a solid phase having an exterior surface including a surface coating layer, the said exterior surface having covalently coupled thereon an inert material to form an undercoating layer on the said exterior surface, or the said exterior surface being coupled to a substantially non-cross-linked, inert material to form an undercoating layer on the said surface, the said undercoating layer being selected to be amenable to mount a binding partner for capturing the said reactant, and the said exterior surface optionally having small pores, which may be filled with the said inert material.

2. An intermediate as claimed in claim 1 wherein the said inert material comprises a proteinaceous material, preferably selected from bovine serum albumin, bovine gamma globulin, apoferritin, ovalbumin, avidin, streptavidin, peptides, polyamino acids and synthetic polyamino compounds.

3. An intermediate as claimed in claim 1 wherein the said inert material comprises a non-proteinaceous material, preferably selected from polyamino compounds and synthetic amino acids.

4. An intermediate as claimed in any of claims 1 to 3 wherein the said inert material is used in an amount sufficient substantially to coat the said solid phase.

5. An intermediate as claimed in any of claims 1 to 4 wherein the said solid phase is selected from magnetic particles, polymer beads, glass beads, titer plates, slides, optical waveguides and test tube walls, which may be silanized, and preferably comprises silanized magnetic metal oxide particles.

6. An intermediate as claimed in any of claims 1 to 5 wherein the said inert material is covalently coupled to the said solid phase via a hetero- or homo-bifunctional cross-linking agent, preferably via glutaraldehyde.

7. An intermediate as claimed in any of claims 1 to 5 wherein the said inert material is directly covalently coupled to the said solid phase without a cross-linking agent.

8. An intermediate as claimed in claim 7 wherein the said inert material and the said solid phase each have functional groups available for peptide bond formation therebetween and the said inert material is directly covalently coupled to the said solid phase using an activating agent selected to activate one of the said functional groups to form a peptide bond, preferably the said solid phase has amino groups available for peptide bond formation and the said inert material has carboxyl groups available for peptide bond formation.

9. An intermediate as claimed in claim 8 wherein the said activating agent is selected from N,N'-dicyclohexylcarbodiimide, N,N'-diisopropylcarbodiimide, 1,1'-carbonyldiimidazole and 1-ethyl-3-(3-dimethylaminopropyl)carbodiimide hydrochloride.

10. A reagent characterised in that it comprises an intermediate as claimed in any of claims 1 to 9 having at least one binding partner attached to the said inert material.

11. A reagent as claimed in claim 10 wherein it comprises a plurality of binding partners attached to the said inert material, each of the said binding partners selected to capture a separate reactant.

12. A reagent as calmed in claim 10 or claim 11 wherein the said at least one binding partner is covalently attached to the said inert material.

13. A reagent as claimed in claim 12 wherein the said inert material and the said binding partner each include functional groups available for covalent coupling selected from carboxyl groups, amine groups, aldehyde groups, hydroxy groups, and solfhydryl groups, the said binding partners being covalently attached to the said inert material using an activating agent, preferably selected from N,N'-dicyclohexylcarbodiimide, N,N'-diisopropylcarbodiimide, 1,1'-carbonyldiimidazole and 1-ethyl-3-(3-dimethylaminopropyl)carbodiimide hydrochloride.

14. A reagent as claimed in claim 10 or claim 11 wherein the said at least one binding partner is non-covalently attached to the said inert material.

15. A reagent as claimed in claim 14 wherein the said at least one binding partner is absorptively attached to the said inert material.

16. A reagent as claimed in any of claims 10 to 15 wherein the said intermediate further comprises a first member of a binding pair coupled to the said undercoating layer, the said first member being selected to bind to a second member of the said pair carrying a binding partner.

17. A reagent as claimed in claim 16 wherein the said binding pair is selected from biotin/avidin and biotin/streptavidin.

18. A reagent as claimed in any of claims 10 to 17 wherein the said binding partner is selected from antibody, antigen, hapten, enzyme, enzyme substrate, inhibitor, cofactor, binding protein, binding protein substrate, carrier protein, carrier protein substrate, lectin, carbohydrate, hormone, hormone receptor, hormone effector, hormone repressor, hormone inducer, peptide, gene probe, avidin, biotin and streptavidin.

19. A reagent as claimed in any of claims 10 to 18 wherein the said inert material is inert with respect to a reactant that forms a complex with the said binding partner.

20. A method of making a reagent capture intermediate or reagent as claimed in any of claims 1 to 19 characterised in that it comprises:
- providing a solid phase having an exterior surface including a surface cotaing layer;
- selecting an inert undercoating material suitable for mounting thereon a binding partner for capturing the said reactant;
- optionally, covalently coupling a cross-linking agent to the said exterior surface, the said inert material being covalently coupled to the said cross-linking agent to form the said undercoating;
- covalently coupling the said inert material to the said exterior surface to form an undercoating thereon;
- optionally, attaching at least one binding partner to the said inert material to form a reagent.

21. A method of capturing at least one reactant characterised in that it comprises:
(a) providing a reagent as claimed in any of claims 10 to 19 including at least one binding partner attached to the said inert material, the said binding partner being capable of forming a complex with the said reactant; and
(b) forming an admixture of the said reagent and a sample containing the said reactant.
